(19)

Europäisches Patentamt

European Patent Office

Office européen des brevets

(11) **EP 4 342 387 A1**

(12) **EUROPEAN PATENT APPLICATION**

(43) Date of publication:
**27.03.2024 Bulletin 2024/13**

(21) Application number: **22197281.3**

(22) Date of filing: **23.09.2022**

(51) International Patent Classification (IPC):
**A61B 8/06** (2006.01) **A61B 8/08** (2006.01)
**A61B 8/00** (2006.01)

(52) Cooperative Patent Classification (CPC):
**A61B 8/54; A61B 8/06; A61B 8/488; A61B 8/5223**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SHULEPOV, Sergei**
  **Eindhoven (NL)**
• **JOSHI, Rohan**
  **Eindhoven (NL)**

(74) Representative: **Philips Intellectual Property & Standards**
**High Tech Campus 52**
**5656 AG Eindhoven (NL)**

(54) **FLUID RESPONSIVENESS USING ULTRASOUND DATA**

(57)    The invention provides an improved measure of fluid responsiveness which is derived based on comparing a ratio between blood velocity and vessel diameter before and after fluid administration.

FIG. 1

EP 4 342 387 A1

**Description**

FIELD OF THE INVENTION

The present invention relates to a method for assessing fluid responsiveness.

BACKGROUND OF THE INVENTION

This invention relates to assessing fluid responsiveness in critically ill patients. Critically ill patients, for instance those in cardiogenic shock or hemorrhagic shock, may benefit from intravenous administration of fluids (e.g., saline, blood) to improve physiological stability and patient outcome. Administering too much fluid however brings with it the risk of volume overload which is detrimental to patient outcomes.

Fluid responsiveness is assessed either after administering fluids or after performing a passive leg raise (PLR), which makes blood from venous circulation in the legs re-enter the arterial circulation. Alterations in cardiac preload provoked due to respiration may also serve as an approach to assess whether the patient is likely to be fluid responsive. There exist several approaches in the art to assessing fluid responsiveness. However, all are based on the same principle of increased stroke volume variation (SVV) or a proxy of SVV upon administration of fluids (or PLR). This is based on the well-known Frank Starling mechanism. A hypovolemic patient is on the steep part of the Frank-Starling curve and will have a larger increase in SVV when administered fluids versus a euvolemic patient.

Many known hemodynamic measurement systems such as PiCCO and FloTrac measure SVV and can thus be used for assessing fluid responsiveness. Instead of using SVV, it is also possible to use changes in stroke volume (SV) or cardiac output (CO) directly, by using hemodynamic measurement devices or via direct imaging of the heart or major vessels (e.g., aorta). It is also possible to use pulse pressure variation (PPV - a proxy for SVV) from the continuously acquired arterial blood pressure signal. The standard approaches for assessing fluid responsiveness are thus dependent on invasive measurements of CO, SV, or blood pressure.

Thus, existing methods for assessing fluid responsiveness rely on approaches that tend to be invasive, indirect, and often unreliable. For instance, PiCCO pulse contour analysis uses changes in blood pressure morphology to determine SVV and is unreliable when assessed at time points far from calibration. FloTrac has the same limitations, and these are persistent as it is an uncalibrated method. Another limitation is that changes in SVV, SV and CO as a marker of fluid responsiveness is defined only for mechanically ventilated patients receiving, for instance, more than 8ml/kg body weight of tidal volume through mechanical ventilation. For patients receiving lower tidal volume or for spontaneously breathing patients, these markers for fluid responsiveness perform poorly.

SUMMARY OF THE INVENTION

The invention is defined by the claims.

According to examples in accordance with an aspect of the invention, there is provided a computer-implemented method, comprising: receiving first ultrasound data of a blood vessel of a subject, the first ultrasound data acquired over a first epoch, the first epoch occurring prior to intra-arterial administration of a bolus of liquid to the subject; receiving second ultrasound data of a blood vessel of a subject, the second ultrasound data acquired over a second epoch, the second epoch occurring following the intra-arterial administration of the bolus of liquid to the subject; determining a first measure of a flow velocity of the blood in the blood vessel of the subject using the first ultrasound data; determining a first measure of a diameter of the blood vessel using the first ultrasound data; determining a second measure of the flow velocity of the blood in the blood vessel of the subject using the second ultrasound data; determining a second measure of the diameter of the blood vessel using the second ultrasound data; determining a first ratio of blood velocity to blood vessel diameter, or a correlate thereof, using the first blood velocity measure and first blood vessel diameter measure; determining a second ratio of blood velocity to blood vessel diameter, or a correlate thereof, using the second blood velocity measure and second blood vessel diameter measure; deriving an index of fluid responsiveness of the subject based on a comparison between the first ratio and second ratio; generating a data output indicative of the index of fluid responsiveness.

Thus the proposed concept is to use a ratio of blood velocity and vessel diameter (or vice versa) as an index of fluid responsiveness. In particular, a comparison of this ratio (e.g. a change in this ratio) between a time point before and a time point after fluid administration is used to derive an index of fluid responsiveness. As will demonstrated later in this document, this index has been found to be both more reliable that at least some existing indices for fluid responsiveness, particularly non-invasive indices, and can be measured non-invasively and quasi-continuously.

The first ratio and second ratio are calculated in the same way; i.e. they are two computations of the same parameter, computed using the same function.

The method may employ use of a pre-defined mapping between ratio comparison results and the fluid responsiveness index.

The artery in some embodiments is preferably the left common carotid artery.

In some embodiments, comparison comprises computing a difference between the first and second ratio.

In some embodiments, each of the first and second ultrasound data include both Doppler ultrasound data and imaging ultrasound data, such as B-mode ultrasound data or M-mode ultrasound data.

In some embodiments, the Doppler ultrasound data is pulsed wave Doppler ultrasound data.

In some embodiments, the first and second blood vessel diameter measures are computed using the imaging ultrasound data in the respective first and second ultrasound data. In some embodiments, the first and second flow velocity measures are computed using the Doppler data in the respective first and second ultrasound data.

In some embodiments, each of the first and second ultrasound data comprise Duplex ultrasound data, the Duplex ultrasound data including interleaved B-mode and Doppler mode acquisitions. This is a highly efficient way of acquiring both types of data in one acquisition process, enabling both the measures to be derived from component elements of the acquired data.

In some embodiments, first and second measure of flow velocity of the blood vessel are a measure of a peak systolic flow velocity (PSV), measured over the respective first and second epochs. As will be explained below, PSV provides a particularly accurate result in the final fluid responsiveness index, comparable with the result obtainable through invasive means.

In some embodiments, the peak systolic flow velocity for each of the first and second flow velocity measures is obtained based on: sampling flow velocity of the blood vessel at multiple time points over the respective epoch, and selecting a flow velocity corresponding to a $90^{th}$ percentile (or approximately/about a $90^{th}$ percentile) of a plot of the sampled velocity over time over the epoch. The inventors propose the $90^{th}$ percentile because it has been found to be particularly robust to noise and motion artifacts.

In some embodiments, the previously mentioned second epoch is timed to begin after a pre-defined time interval following the administration of the bolus. The computation of the fluid responsiveness index may assume this time interval.

In some embodiments, said pre-defined time interval following the administration of the bolus is approximately 15 minutes.

In some embodiments, each epoch has a duration of 120 seconds or less, for example 60 seconds or less, for example between about 20 seconds and about 60 seconds. Thus this approach proposes measuring epochs before and after the fluid bolus and comparing them to obtain a discrete measure of the fluid responsiveness index.

It is also possible to measure the index continuously (or quasi-continuously) using a moving window starting after the bolus has been administered and comparing the ratio value computed over this window against the pre-bolus ratio value, thus obtaining a continuous or quasi-continuous fluid responsiveness index measurement.

To this end, in some embodiments, the method comprises repeating at regular time intervals the said steps of: receiving the second ultrasound data; determining the second measure of the flow velocity; determining the second measure of the diameter of the blood vessel; determining the second ratio of blood velocity to blood vessel diameter; and deriving an index of fluid responsiveness of the subject, so as to provide post-bolus monitoring of the fluid responsiveness index. This therefore provides repeated or recurring measurements.

The method may comprise recording a log of each derived value of the index of fluid responsiveness, along with a measurement time to which it corresponds, and determining trend information of the index of fluid responsiveness as a function of time, and generating a data output indicative of the trend information.

In some embodiments, the first and second ultrasound data are received from an ultrasound acquisition apparatus.

In some embodiments, the method comprises: generating first control instructions for communication to an ultrasound acquisition apparatus to cause the apparatus to acquire the first ultrasound data; and generating second control instructions for communication to the ultrasound acquisition apparatus to cause the apparatus to acquire the second ultrasound data.

In some embodiments, the method further comprises receiving a user input indicating a timing of the administration of the bolus of liquid, and wherein the second control instructions are generated so as to cause the ultrasound acquisition apparatus to acquire the second ultrasound data at a time determined based on the said user input.

Another aspect of the invention is a computer program product comprising computer code configured to cause a processor to perform a method in accordance with any embodiment described in this document, or in accordance with any claim of this application.

The invention can also be embodied in hardware form.

Thus, another aspect of the invention is a processing unit, comprising: an input/output; and one or more processors, configured to: receive, via the input/output, first ultrasound data of a blood vessel of a subject, the first ultrasound data acquired over a first epoch, the first epoch occurring prior to intra-arterial administration of a bolus of liquid to the subject; receiving, via the input/output, second ultrasound data of a blood vessel of a subject, the second ultrasound data acquired over a second epoch, the second epoch occurring following the intra-arterial administration of the bolus of liquid to the subject; determine a first measure of a flow velocity of the blood in the blood vessel of the subject using the first ultrasound data; determine a first measure of a diameter of the blood vessel using the first ultrasound data; determining a second measure of the flow velocity of the blood vessel of the subject using the second ultrasound data; determine a second measure of the diameter of the blood vessel using the second ultrasound data; determine a first ratio of blood velocity to blood vessel diameter, or a correlate thereof, using the first blood velocity measure and first blood vessel diameter measure; determine a second ratio of blood velocity to blood vessel diameter, or a correlate thereof, using the second blood velocity measure and second blood vessel diameter measure; derive an index of fluid responsiveness of the subject based on a comparison between the first ratio and second ratio; and generate a data output indicative of the index of fluid responsiveness, and preferably routing the generated data output to the input/output.

Another aspect of the invention provides a system, comprising: a processing unit as outlined above, or in accordance with any embodiment described in this document, and an ultrasound acquisition apparatus operatively coupled with the processing unit. The first and second ultrasound data are received from the ultrasound acquisition apparatus.

In some advantageous embodiments, the ultrasound acquisition apparatus comprises a wearable ultrasound transducer unit, for example a wearable patch integrating an ultrasound transducer arrangement. This allows for continuous, non-invasive monitoring in a way that is efficient and non-intrusive.

Further to the above, it is noted that there is intended to be complete compatibility in features between the different aspects of the invention (system, method, computer program product), so that features or options recited in the context of one aspect can be applied equally to any other aspect.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 schematically outlines components of an example processing arrangement and system in accordance with one or more embodiments of the invention;
Fig. 3 illustrates timings of data acquisition relative to a timing of a fluid bolus administration; and
Figs. 4-11 illustrate results of an experimental test demonstrating relative efficacy of different indices of fluid responsiveness, wherein Fig. 11 shows the results for the ratio of velocity:diameter proposed in accordance with embodiments of the present invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an improved measure of fluid responsiveness which is derived based on comparing a ratio between blood velocity and vessel diameter before and after fluid administration.

Maintaining adequate fluid balance is critical in optimizing outcomes in critically ill patients. To this end, clinicians have a need to identify whether a patient is fluid responsive before administering a large fluid bolus to avoid fluid overload. Existing methods to measure fluid responsiveness are typically invasive, indirect and often unreliable. Proposed herein is a dynamic index derivable from diameter and velocity measurements of an artery by using ultrasound. This method is a non-invasive and is a direct measure of fluid responsiveness.

Particularly advantageous embodiments may optionally employ use of a wearable ultrasound acquisition means. This provides the further improvement of non-intrusiveness and also increases operator independence of measurements since a wearable ultrasound unit can be affixed to a subject at a stable arterial location.

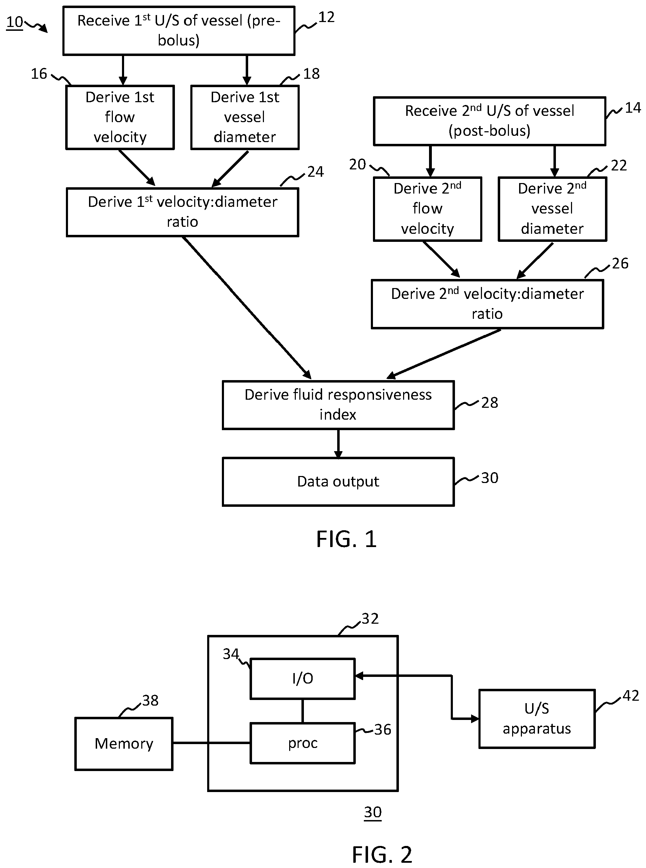

Fig. 1 outlines in block diagram form steps of an example method according to one or more embodiments. The steps will be recited in summary, before being explained further in the form of example embodiments.

The method can be implemented by a computer.

The method 10 comprises receiving 12 first ultrasound data of a blood vessel of a subject, the first ultrasound data acquired over a first epoch, the first epoch occurring prior to intra-arterial administration of a bolus of liquid to the subject. The method 10 further comprises receiving 14 second ultrasound data of a blood vessel of a subject, the second ultrasound data acquired over a second epoch, the second epoch occurring following the intra-arterial administration of the bolus of liquid to the subject. The blood vessel may for example be a peripheral blood vessel, e.g. a peripheral artery, for maximal ease of measurement.

The method 10 further comprises determining 16 a first measure of a flow velocity of the blood in the blood vessel of the subject, or a correlate thereof, using the first ultrasound data. The method 10 further comprises determining 18 a first measure of a diameter of the blood vessel using the first ultrasound data.

The method 10 further comprises determining 20 a second measure of the flow velocity of the blood in the blood vessel of the subject, or a correlate thereof, using the second ultrasound data. The method 10 further comprises determining 22 a second measure of the diameter of the blood vessel using the second ultrasound data.

The method 10 further comprises determining 24 a first ratio of blood velocity to blood vessel diameter, or a correlate thereof, using the first blood velocity measure and first blood vessel diameter measure. The method 10 further comprises determining 26 a second ratio of blood velocity to blood vessel diameter, or a correlate thereof, using the second blood velocity measure and second blood vessel diameter measure

The method further comprises deriving 28 an index of fluid responsiveness of the subject based on a comparison between the first ratio and second ratio. The method may further comprise generating 30 a data output indicative of the index of fluid responsiveness. For example the data output may be communicated to a datastore for storage or to a further computer for use in further processing. The data output may be communicated to a user interface for presentation to user through the user interface.

Thus, embodiments of the present invention provide as inputs: the arterial diameter and blood velocity (both obtained from ultrasound data) to a novel algorithm that translates these measurements into an index of fluid responsiveness (based on a ratio between the two, or a correlate thereof). This software feature can be enabled on any ultrasound system (including among others wearable and handheld) to non-invasively assess fluid responsiveness.

It is noted that although the above description indicates measuring a diameter of the vessel, clearly measuring a parameter which is correlated or proportional to this achieves an entirely equivalent effect, e.g. measuring radius or circumference, would achieve a same effect.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system does not have to comprise all of the illustrated hardware components; it may just comprise a subset of them.

The processing unit 32 comprises one or more processors 36 configured to perform a method in accordance with that outlined above, or in accordance with any embodiment described in this document or any claim of this application. In the illustrated example, the processing unit further comprises an input/output 34 or communication interface. In the illustrated example of Fig. 2, the system 30 further comprises an ultrasound acquisition apparatus 42 operatively coupled with the processing unit 32. The previously mentioned first and second ultrasound data may be received from the ultrasound acquisition apparatus. Alternatively, the first and second ultrasound data might be received from a different source, such as from a datastore, or from a central server, or from a communication interface. In some embodiments, the ultrasound data may be received from an ultrasound acquisition apparatus which is external to the provided system.

The invention can also be embodied in software form. Thus, another aspect of the invention is a computer program product comprising computer program code configured, when run on a processor, to cause the processor to perform the method in accordance with embodiment described in this document, or in accordance with any claim of this application.

It is noted, with reference again to the system 30 of Fig. 2, that the system 30 or the processing unit 32 may include a memory 38 which stores computer program instructions for execution by the one or more processors 36 of the processing unit to cause the one or more processors to perform a method in accordance with any of the embodiments described in this disclosure.

In some embodiments, the method 10 may comprise steps for controlling acquisition of the first and second ultrasound data. For example, the method 10 may comprise generating first control instructions for communication to an ultrasound acquisition apparatus 42 to cause the apparatus to acquire the first ultrasound data; and generating second control instructions for communication to the ultrasound acquisition apparatus to cause the apparatus to acquire the second ultrasound data.

In some embodiments, the method further comprises receiving a user input indicating a timing of the administration of the bolus of liquid, and wherein the second control instructions are generated so as to cause the ultrasound acquisition apparatus to acquire the second ultrasound data at a time determined based on the said user input. This could be received for instance from a user interface device (not shown).

With regards to the ultrasound data acquisition apparatus 42, in advantageous embodiments, this may comprise a wearable ultrasound transducer unit, for example a wearable patch integrating an ultrasound transducer arrangement. For instance, the wearable unit may carry an ultrasound transducer array. A wearable patch may have an adhesion element to permit removable adhesion of the patch to the skin of the user in a manner that renders the ultrasound transducer array in sonic coupling with the body tissue. Other forms of wearable unit are also possible, such as a band which can be worn by the user, or a garment with an integrated transducer arrangement. In operation the transducer unit can be coupled to the body of the user in position above a blood vessel of interest, for example a peripheral artery, for example the carotid artery, for example the left common carotid artery. Use of wearable ultrasound may facilitate computation of the index of fluid-responsiveness in a manner that is both continuous and non-invasively obtained.

With regards to the first 12 and second 14 ultrasound data, it is preferred that this ultrasound data in each case comprises both Doppler ultrasound data and imaging ultrasound data, such as B-mode ultrasound data. The Doppler ultrasound data may be pulsed wave Doppler ultrasound data, but this is not essential. The Doppler data can be used to measure the blood flow velocity through the vessel. The imaging data, e.g. B-mode data, can be used to measure the diameter of the blood vessel. In other words, the first and second blood vessel diameter measures may computed using the imaging ultrasound data in the respective first and second ultrasound data and the first and second flow velocity measures may be computed using the Doppler data in the respective first and second ultrasound data.

By 'imaging data' is meant acquisition of line-scan data which is suitable for use in reconstructing an image. For example, another way of saying this is multi-line data. Another way of saying this is that the ultrasound data includes scan line data spanning at least one plane. The determining of the vessel diameter could be performed by actually reconstructing an image from the acquired imaging data, or it could be done through a more low-level method, based for instance on detecting characteristic intensity points in the acquired ultrasound lines which are taken to correspond to boundaries of the vessel, and computing a distance between these points.

A convenient and technically efficient way of acquiring both Doppler ultrasound data and B-mode ultrasound data is to use Duplex ultrasound imaging. Thus, in some embodiments, each of the first and second ultrasound data comprise Duplex ultrasound data, the Duplex ultrasound data including interleaved B-mode and Doppler mode acquisitions. The skilled person will know means for implementing Duplex imaging.

The invention can also be embodied in software form. The invention can also be embodied in software. Thus, another aspect of the invention is a computer program product comprising computer program code means configured, when run on a processor, to cause the processor to perform the method in accordance with embodiment described in this document, or in accordance with any claim of this application.

Embodiments of the invention include steps of measuring blood flow velocity through a vessel before and after administration of fluid. For this purpose, it is proposed in a preferred set of embodiments to measure a value of peak systolic blood velocity (PSV) over one or more heart cycles spanning the respective measurement epoch. For example, a mean value of peak systolic velocity (PSV) might be computed from multiple heart beats. For consistency, likewise a mean value of vessel diameter might be measured over the same multiple heartbeats. The ratio of blood velocity to blood vessel diameter might then be computed as a ratio of the mean values.

In some instances, the raw PSV may be prone to errors in measurement. Thus, in some embodiments, to improve robustness, a correlate of PSV may be measured. For example, in some embodiments, the method comprises using a 90th percentile of the blood flow velocity over the relevant measurement epoch. In other words, to put this more concretely, in some embodiments, the peak systolic flow velocity (PSV) for each of the first and second flow velocity measures may be obtained based on: sampling flow velocity of the blood vessel at multiple time points over the respective epoch; and selecting a flow velocity corresponding to a 90th percentile of a plot of the sampled velocity over time over the epoch.

Furthermore, it is noted that instead of taking the ratio between velocity and diameter, the inverse could instead be taken, which achieves the same effect.

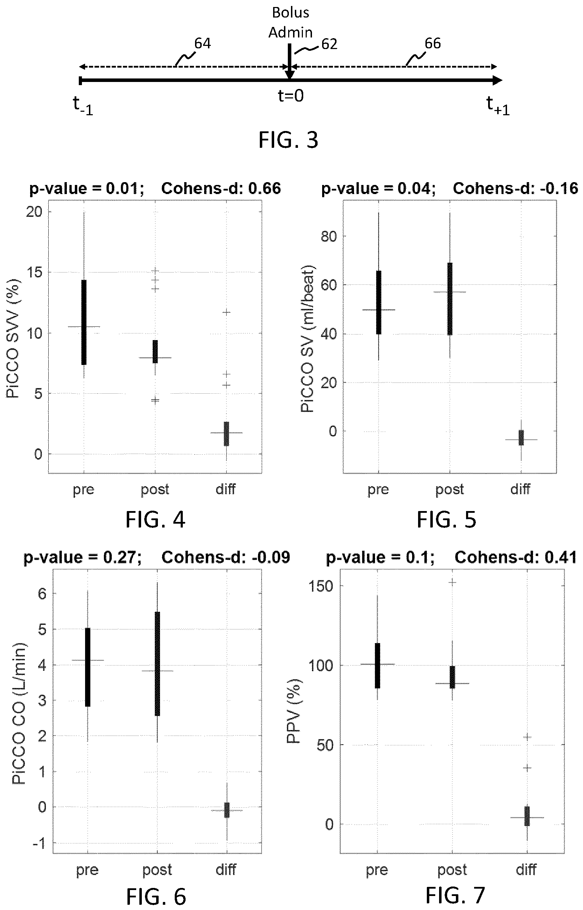

The timing scheme of the method is schematically illustrated in Fig. 3. The intra-arterial administration of a bolus of liquid to the subject is shown at nominal time t=0. Before the bolus administration is a pre-bolus time period, which is indicated as starting at nominal time $t_{-1}$. After the bolus administration is a post-bolus period, which is indicated as ending at post-bolus time $t_{+1}$. By way of example, the pre-bolus period and post-bolus period might each have a duration of 15 minutes. Within each of these periods, at least one measurement of the blood vessel diameter of blood flow velocity is taken, the measurement obtained using ultrasound data acquired over a respective measurement epoch. A single measurement epoch would typically be short compared to whole duration of the pre- or post- bolus period. In practice, multiple measurements might be taken in each of the pre- and pots-bolus periods, each based on ultrasound data acquired over a respective measurement epoch. By way of example, for each measurement of vessel diameter and blood flow velocity, ultrasound data might be acquired over an ultrasound epoch of e.g. 20-60s duration (this is just an example for illustration). If more than one measurement is to be taken before and/or after fluid administration, then a mean of the multiple measured diameters and flow velocities might be taken and used to compute a single overall ratio for each of the pre- and post- fluid administration periods.

To demonstrate the efficacy of the proposed metric as an indicator of fluid responsiveness, experimental data was acquired based on a test procedure which will now be described, with reference to Figs. 4-11 which show statistical results of the test.

The test was performed with a test cohort of 13 patients, with 14 fluid boluses administered in total in an operating room setting.

In accordance with the scheme already described above with reference to Fig. 3, a fluid bolus 62 was administered for each patient at nominal time t = 0. In 15 minute periods before 64 and after 66 bolus administrations, ultrasound data was acquired over ultrasound measurement epochs of 20-60s duration, obtained for the test procedure using a handheld ultrasound probe. In addition, for purposes of comparison, arterial blood pressure (ABP) measurements and PiCCO-based stroke volume variation (SVV), stroke volume (SV) and cardiac output (CO) measurements were obtained. The ABP measurements were acquired through invasive measurement means, and the SVV, SV and CO measurements were also all acquired invasively.

These were only needed for the experimental test, for purposes of validation of the fluid responsiveness index proposed in accordance embodiments of the present invention, as will become clear.

There was performed at least one ultrasound measurement in both the pre- 64 and post-66 bolus time periods. If more than one ultrasound measurement epoch was conducted, the mean of the ultrasound-derived measurement over the whole period 64, 66, was taken. In effect the pre-bolus period acts as the control period while the post-bolus period serves as the test period.

The measures of ABP, SVV, SV and CO were obtained as comparative measures. As was explained above, stroke volume variation, or proxy thereof, is the standard parameter used to measure fluid responsiveness. This is based on the well-known Frank Starling mechanism. A hypovolemic patient is on the steep part of the Frank-Starling curve and will have a larger increase in SVV when administered fluids versus a euvolemic patient.

Stroke volume (SV) and cardiac output (CO) can also be used as indirect measures of fluid responsiveness. It is also possible to use pulse pressure variation (PPV - a proxy for SVV) from the continuously acquired arterial blood pressure signal.

The results of the obtained data for the test procedure are presented in Figs. 4-11. Each of the presented charts relates to data for one measured parameter (described below). For each parameter, three values are plotted: the value of the parameter measured pre-fluid administration ("Pre"), the value of the parameter measured post-fluid administration ("post"), and the difference between the two ("diff'). Also shown for each graph are two statistical values which seek to represent the statistical significance of the observed difference between the pre- and post- values and the statistical magnitude of the observed difference. This effectively gives an indication of the efficacy of the respective parameter in representing the real effect of change in fluid responsiveness. The statistical values in particular are the p-values and the Cohen's-d values. The p-values are paired t-tests while Cohen's-d is a measure of effect size. These are standard statistical measurements used to validate a hypothesis against observed data. A p-value measures the probability of obtaining the observed results, assuming that the null hypothesis is true. The lower the p-value, the greater the statistical significance of the observed difference. The Cohen's d-value measures the statistical size of the effect.

Thus in summary, the charts presented in Figs. 4-11 allow for comparison of the efficacy of the different measured parameters as indicators/metrics of fluid responsiveness. The lower the p-value, and the higher the Cohen's-d value, the more robust the parameter as an indicator fluid responsiveness. Since SVV is the standard parameter used for assessing fluid responsiveness, it provides a very valuable baseline against which other parameters can be compared.

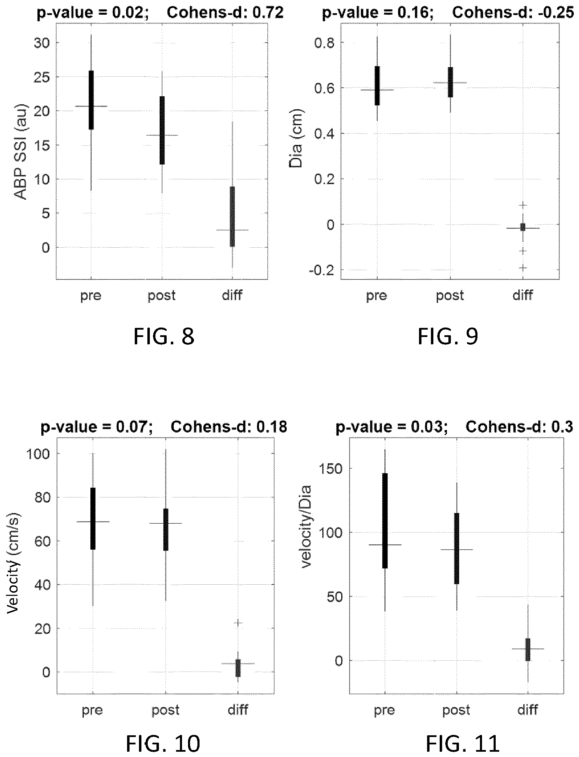

Of most importance in the present context is Fig. 11 which shows the results for the proposed fluid responsiveness index based on ratio of blood velocity and vessel diameter.

In summary, the graphs relate to following parameters. Fig. 4 shows results for SVV (units: %). Fig. 5 shows results for SV (units: ml/beat). Fig. 6 shows results for CO (units: L/min). Fig. 7 shows results for PPV (units: %). Fig. 8 shows results for ABP SSI (see below for explanation) (units: au). Fig. 9 shows results for diameter ("Dia") of blood vessel (units: cm). Fig. 10 shows results for blood flow velocity ("velocity"), in particular peak systolic velocity (units: cm/s). Fig. 11 shows results for the ratio of peak velocity to vessel diameter.

The parameters SVV, SV and CO were obtained from a PiCCO device. The parameters PPV and ABP SSI were calculated from an invasively measured ABP waveform. The parameters Dia, velocity and velocity/Dia were derived from obtained ultrasound data in the manner already described above.

More particularly, PPV was calculated using the ABP. Traditionally, PPV is defined as follows:

$$PPV\% = \frac{ABP(max) - ABP(min)}{ABP(mean)} * 100$$

Here, ABP (max), ABP (min) and ABP (mean) refers the maximum, minimum and mean value of the ABP over the measurement epoch. For the purposes of the experiment, PPV was computed in a similar manner, but with the maximum and minimum values replaced with instead the 99th and 1st percentile values of the ABP, since these are more robust to noise.

ABP SSI is a novel ABP-based feature calculated as follows:

$$ABP\ SSI = \frac{99th\ percentile\ of\ ABP - 75th\ percentile\ of\ ABP}{SSI} * 100$$

Here, signal stability index (SSI) is an index previously defined in document EP3571991A1. By way of brief summary, the SSI is a non-parametric method based on the kernel density estimate for determining the probability density function of an underlying signal. The probability density is approximated by the superposition of a number of Gaussian kernels centred on a number of equidistant points (here 100 points) on a segment of the signal. The maximum value of the superposition of these Gaussian kernels forms the SSI. The SSI is high if the mean and variance of the underlying Gaussian kernels are comparable. The ABP SSI feature performs better than the traditional PPV measure and is novel.

'Dia' and 'velocity' are the diameter and peak systolic velocity derived from the US B-mode and PWD signals respectively. The 'velocity/Dia' is the ratio of the 90th percentile value of the velocity waveform divided by the mean value of the diameter over the measurement epoch. This is the metric of fluid responsiveness which is proposed in accordance with embodiments of the present invention.

Reviewing Fig. 11, which relates to the proposed ratio velocity/diameter, the p-value was 0.03 and Cohen's -d value was 0.3. It is clear from the results that this metric performs better than the diameter of the peak systolic velocity by itself (which obtained a p-value of 0.16 and Cohen's -d value of -0.25), and better than the blood flow velocity by itself (p-value =0.07 and Cohen's =-d 0.18). It is also comparable with the results for the classical SSV parameter (p-value =0.01 and Cohen's -d =0.66) and SV parameter (p-value =0.04 and Cohen's -d =-0.16). Indeed, the proposed ratio performed an order of magnitude better, in terms of p-value, than the classical parameters of CO (p-value =0.27 and Cohen's -d =-0.09) and PPV (p-value =0.1 and Cohen's -d =0.41).

Thus, the results demonstrate the reliability of the proposed ratio of flow velocity : diameter as a basis for computing a fluid responsiveness index. Furthermore, the proposed ratio can be measured fully non-invasively and very rapidly.

The ultrasound-based feature calculation proposed by the inventors differs from the calculation of SVV, SV, CO, and ABP-based features since it can be calculated using data acquired over only short measurement epochs of e.g. 20-60s in the pre- and post-bolus period, whereas the other features are calculated over continuous 15 min measurement epochs. This makes it quicker and easier to obtain measurements.

Indeed, quasi-continuous measurement is also a possibility with continuous ultrasound monitoring techniques, e.g. a wearable ultrasound transducer unit. Further, if the measurements are made with a wearable ultrasound device, repeatability errors could be substantially mitigated, further improving performance.

It is also noted that the fluid responsiveness metric proposed in accordance with the present invention is not specifically utilizing respiratory modulation and, for this reason, it is broadly applicable to both ventilated and spontaneously breathing patients.

The same ratio may indeed applicable beyond fluid responsiveness assessment, to determine the efficacy of and for titrating the administration of vasodilators and vasoconstrictors.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing unit can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method, comprising:

   receiving (12) first ultrasound data of a blood vessel of a subject, the first ultrasound data acquired over a first epoch, the first epoch occurring ($t\_-1$) prior to intra-arterial administration of a bolus of liquid to the subject;
   receiving (14) second ultrasound data of a blood vessel of a subject, the second ultrasound data acquired over a second epoch, the second epoch occurring ($t\_+1$) following the intra-arterial administration of the bolus of liquid to the subject;
   determining (16) a first measure of a flow velocity of the blood in the blood vessel of the subject using the first ultrasound data;
   determining (18) a first measure of a diameter of the blood vessel using the first ultrasound data;
   determining (20) a second measure of the flow velocity of the blood in the blood vessel of the subject using the second ultrasound data;
   determining (22) a second measure of the diameter of the blood vessel using the second ultrasound data;
   determining (24) a first ratio of blood velocity to blood vessel diameter, or a correlate thereof, using the first blood velocity measure and first blood vessel diameter measure;
   determining (26) a second ratio of blood velocity to blood vessel diameter, or a correlate thereof, using the second blood velocity measure and second blood vessel diameter measure;
   deriving (28) an index of fluid responsiveness of the subject based on a comparison between the first ratio and second ratio;
   generating (30) a data output indicative of the index of fluid responsiveness.

2. The method of claim 1, wherein the comparison comprises computing a difference between the first and second ratio.

3. The method of claim 1 or 2, wherein each of the first and second ultrasound data include both Doppler ultrasound data and imaging ultrasound data, such as B-mode ultrasound data, and optionally wherein the Doppler ultrasound data is pulsed wave Doppler ultrasound data.

4. The method of claim 3,

   wherein the first and second blood vessel diameter measures are computed using the imaging ultrasound data in the respective first and second ultrasound data; and
   wherein the first and second flow velocity measures are computed using the Doppler data in the respective first and second ultrasound data.

5. The method of any of claims 3-4, wherein each of the first and second ultrasound data comprise Duplex ultrasound data, the Duplex ultrasound data including interleaved B-mode and Doppler mode acquisitions.

6. The method of any of claims 1-5, wherein each of the first and second measure of flow velocity of the blood vessel are a measure of a peak systolic flow velocity (PSV), measured over the respective first and second epochs.

7. The method of claim 6, wherein the peak systolic flow velocity for each of the first and second flow velocity measures is obtained based on:

sampling flow velocity of the blood vessel at multiple time points over the respective epoch, and
selecting a flow velocity corresponding to a 90th percentile of a plot of the sampled velocity over time over the epoch.

8. The method of any of claims 1-7, wherein the second epoch is timed to begin after a pre-defined time interval following the administration of the bolus, and optionally wherein said pre-defined time interval following the administration of the bolus is approximately 15 minutes.

9. The method of any of claims 1-8, wherein each epoch has a duration of 120 seconds or less, for example 60 seconds or less, for example between about 20 seconds and about 60 seconds.

10. The method of any of claims 1-9, wherein the method comprises:
repeating at regular time intervals the said steps of:

receiving the second ultrasound data,
determining the second measure of the flow velocity;
determining the second measure of the diameter of the blood vessel,
determining the second ratio of blood velocity to blood vessel diameter, and
deriving an index of fluid responsiveness of the subject,
so as to provide post-bolus monitoring of the fluid responsiveness index.

11. The method of any of claims 1-10, wherein the method comprises:

generating first control instructions for communication to an ultrasound acquisition apparatus to cause the apparatus to acquire the first ultrasound data, and
generating second control instructions for communication to the ultrasound acquisition apparatus to cause the apparatus to acquire the second ultrasound data, and
optionally wherein the method further comprises receiving a user input indicating a timing of the administration of the bolus of liquid, and wherein the second control instructions are generated so as to cause the ultrasound acquisition apparatus to acquire the second ultrasound data at a time determined based on the said user input.

12. A computer program product comprising computer code configured to cause a processor to perform a method in accordance with any of claims 1-11.

13. A processing unit (32), comprising:

an input/output (34); and
one or more processors (36), configured to:

receive, via the input/output, first ultrasound data of a blood vessel of a subject, the first ultrasound data acquired over a first epoch, the first epoch occurring (t_-1) prior to intra-arterial administration of a bolus of liquid to the subject;
receive, via the input/output, second ultrasound data of a blood vessel of a subject, the second ultrasound data acquired over a second epoch, the second epoch occurring (t_+1) following the intra-arterial administration of the bolus of liquid to the subject;
determine a first measure of a flow velocity of the blood in the blood vessel of the subject using the first ultrasound data;
determine a first measure of a diameter of the blood vessel using the first ultrasound data;
determine a second measure of the flow velocity of the blood vessel of the subject using the second ultrasound data;
determine a second measure of the diameter of the blood vessel using the second ultrasound data;
determine a first ratio of blood velocity to blood vessel diameter, or a correlate thereof, using the first blood velocity measure and first blood vessel diameter measure;
determine a second ratio of blood velocity to blood vessel diameter, or a correlate thereof, using the second blood velocity measure and second blood vessel diameter measure;
derive an index of fluid responsiveness of the subject based on a comparison between the first ratio and second ratio; and
generate a data output indicative of the index of fluid responsiveness and routing the generate data output to the input/output.

**14.** A system (30), comprising:

the processing unit (32) of claim 13; and
an ultrasound acquisition apparatus (42) operatively coupled with the processing unit,
wherein the first and second ultrasound data are received from the ultrasound acquisition apparatus.

**15.** The system of claim 14, wherein the ultrasound acquisition apparatus comprises a wearable ultrasound transducer unit, for example a wearable patch integrating an ultrasound transducer arrangement.

10 ⟋

| Receive 1st U/S of vessel (pre-bolus) | ⟋ 12 |

16 ⟋

| Derive 1st flow velocity | Derive 1st vessel diameter | ⟋ 18 |

| Derive 1st velocity:diameter ratio | ⟋ 24 |

| Receive 2nd U/S of vessel (post-bolus) | ⟋ 14 |

20 ⟋

| Derive 2nd flow velocity | Derive 2nd vessel diameter | ⟋ 22 |

| Derive 2nd velocity:diameter ratio | ⟋ 26 |

| Derive fluid responsiveness index | ⟋ 28 |

| Data output | ⟋ 30 |

## FIG. 1

32 ⟋

34 ⟋

| I/O |

38 ⟋

| Memory |

| proc | ⟋ 36 |

42 ⟋

| U/S apparatus |

30

## FIG. 2

Bolus
Admin
64    62    66

$t_{-1}$    t=0    $t_{+1}$

FIG. 3

p-value = 0.01;   Cohens-d: 0.66

FIG. 4

p-value = 0.04;   Cohens-d: -0.16

FIG. 5

p-value = 0.27;   Cohens-d: -0.09

FIG. 6

p-value = 0.1;   Cohens-d: 0.41

FIG. 7

FIG. 8

FIG. 9

FIG. 10

FIG. 11

Europäisches
Patentamt

European
Patent Office

Office européen
des brevets

# EUROPEAN SEARCH REPORT

Application Number

EP 22 19 7281

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MONNET XAVIER ET AL: "Measuring aortic diameter improves accuracy of esophageal Doppler in assessing fluid responsiveness", CRITICAL CARE MEDICINE., vol. 35, no. 2, 1 February 2007 (2007-02-01), pages 477-482, XP93028286, US ISSN: 0090-3493, DOI: 10.1097/01.CCM.0000254725.35802.17 Retrieved from the Internet: URL:http://dx.doi.org/10.1097/01.CCM.00002 54725.35802.17> * abstract * | 1-15 | INV. A61B8/06 A61B8/08 A61B8/00 |
| A | LU NIANFANG ET AL: "Exploring the best predictors of fluid responsiveness in patients with septic shock", AMERICAN JOURNAL OF EMERGENCY MEDICINE, CENTRUM PHILADELPHIA, PA, US, vol. 35, no. 9, 22 March 2017 (2017-03-22) , pages 1258-1261, XP085166549, ISSN: 0735-6757, DOI: 10.1016/J.AJEM.2017.03.052 * abstract * | 1-15 | |
| A | BUCCI M ET AL: "Respiratory variation in aortic blood peak velocity and caudal vena cava diameter can predict fluid responsiveness in anaesthetised and mechanically ventilated dogs", VETERINARY JOURNAL, vol. 227, September 2017 (2017-09), pages 30-35, XP085234486, ISSN: 1090-0233, DOI: 10.1016/J.TVJL.2017.08.004 * abstract * | 1-15 | |

TECHNICAL FIELDS
SEARCHED       (IPC)

A61B

-/--

The present search report has been drawn up for all claims

| Place of search | Date of completion of the search | Examiner |
|---|---|---|
| Munich | 3 March 2023 | Mundakapadam, S |

EPO FORM 1503 03.82 (P04C01)

page 1 of 2

Europäisches
Patentamt

European
Patent Office

Office européen
des brevets

# EUROPEAN SEARCH REPORT

Application Number

EP 22 19 7281

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/353157 A1 (EIBL JOSEPH [CA] ET AL) 13 December 2018 (2018-12-13) * paragraphs [0002], [0115] - [0302]; claims; figures * | 1-15 | |
| A | US 2020/163650 A1 (KRUGER GRANT [US] ET AL) 28 May 2020 (2020-05-28) * paragraphs [0018] - [0079] * | 1-15 | |
| A | LAHER ABDULLAH E ET AL: "A review of hemodynamic monitoring techniques, methods and devices for the emergency physician", AMERICAN JOURNAL OF EMERGENCY MEDICINE, CENTRUM PHILADELPHIA, PA, US, vol. 35, no. 9, 18 March 2017 (2017-03-18) , pages 1335-1347, XP085166538, ISSN: 0735-6757, DOI: 10.1016/J.AJEM.2017.03.036 * abstract * | 1-15 | |
| A | MONNET XAVIER ET AL: "Measuring aortic diameter improves accuracy of esophageal Doppler in assessing fluid responsiveness", CRITICAL CARE MEDICINE., vol. 35, no. 2, 1 February 2007 (2007-02-01), pages 477-482, XP093028286, US ISSN: 0090-3493, DOI: 10.1097/01.CCM.0000254725.35802.17 Retrieved from the Internet: URL:http://dx.doi.org/10.1097/01.CCM.00002 54725.35802.17> * abstract * | 1-15 | **TECHNICAL FIELDS SEARCHED (IPC)** |

The present search report has been drawn up for all claims

| Place of search | Date of completion of the search | Examiner |
|---|---|---|
| Munich | 3 March 2023 | Mundakapadam, S |

EPO FORM 1503 03.82 (P04C01)

## ANNEX TO THE EUROPEAN SEARCH REPORT
## ON EUROPEAN PATENT APPLICATION NO.

EP 22 19 7281

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2023

| Patent document cited in search report | Publication date | Patent family member(s) | Publication date |
|---|---|---|---|
| US 2018353157 A1 | 13-12-2018 | US 2018353157 A1<br>US 2021290201 A1 | 13-12-2018<br>23-09-2021 |
| US 2020163650 A1 | 28-05-2020 | US 2020163650 A1<br>WO 2018209140 A1 | 28-05-2020<br>15-11-2018 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3571991 A1